(19) Europäisches Patentamt
European Patent Office
Office européen des brevets

(11) Publication number: **0 245 901 B1**

# (12) EUROPEAN PATENT SPECIFICATION

(45) Date of publication of patent specification: **26.08.92**

(51) Int. Cl.[5]: **C07D 279/06**, C07D 417/12, A01N 43/86

(21) Application number: **87200817.2**

(22) Date of filing: **29.04.87**

## (54) Thiazinone derivatives.

(30) Priority: **13.05.86 GB 8611617**

(43) Date of publication of application:
**19.11.87 Bulletin 87/47**

(45) Publication of the grant of the patent:
**26.08.92 Bulletin 92/35**

(84) Designated Contracting States:
**AT BE CH DE ES FR GB GR IT LI LU NL SE**

(56) References cited:
**DD-A- 108 541**
**GB-A- 2 140 010**

**CHEMICAL ABSTRACTS, vol. 83, no. 9, 1st September 1975, page 667, abstract no. 79264j, Columbus, Ohio, USA**

**JUSTUS LIEBIGS ANN. CHEM., vol. 77, no. 8, pages 1249-1266**

**CAN. J. CHEM., vol. 59, no. 14, pages 2223-2227**

**AUST. J. CHEM., vol. 23, no. 1, pages 51-72**

**CHEMICAL ABSTRACTS, vol. 94, abstract-no. 78267c**

**CHEMICAL ABSTRACTS, vol. 68, abstract-no. 59513b**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Gilkerson, Terence**
**11 The Ness**
**Canterbury Kent CT1 3NL(GB)**
Inventor: **Jennens, David Clifford**
**13 Thistle Walk**
**Sittingbourne Kent(GB)**
Inventor: **Coombs, Mandy Elizabeth**
**17 Meadow Rise Iwade**
**Sittingbourne Kent ME9 8SB(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to thiazinone derivatives, a process for the preparation of such compounds, compositions containing them, and to their use as fungicides.

Justus Liebigs Ann. Chem., 77(8), pp. 1249-66 discloses 2-phenylamino-1,3-thiazin-4-one, 2-(2-methoxyphenylamino)-1,3-thiazin-4-one, 2-methylamino-1,3-thiazin-4-one, 2-tert-butylamino-1,3-thiazin-4-one, 2-methoxy-1,3-thiazin-4-one, 2-phenylamino-5,6-dihydro-1,3-thiazin-4-one and 2-tert-butylamino-5,6-dihydro-1,3-thiazin-4-one. Can. J. Chem., (1981), 59(14), pp. 2223-7 and Aust. J. Chem., (1970), 23 (1), pp. 51-72 both disclose 2-methylthio-1,3-thiazin-4-one. DD 108541 also discloses 2-phenylamino-5,6-dihydro-1,3-thiazin-4-one. Chem. Abs. 68: 59513b discloses 2-phenylamino-5,6-dihydro-1,3-thiazin-4-one, 2-(4-nitrophenylamino)-5,6-dihydro-1,3-thiazin-4-one, 2-(4-methylphenylamino)-5,6-dihydro-1,3-thiazin-4-one, 2-(2-methylphenylamino)-5,6-dihydro-1,3-thiazin-4-one, 2-(4-chlorophenylamino)-5,6-dihydro-1,3-thiazin-4-one, 2-(α-naphthylamino)-5,6-dihydro-1,3-thiazin-4-one, 2-(β-naphthylamino)-5,6-dihydro-1,3-thiazin-4-one and 2-(2-methoxyphenylamino)-5,6-dihydro-1,3-thiazin-4-one. However, none of these documents refer to or suggest any fungicidal activity for these compounds.

Chem. Abs. 94: 78267c also discloses 2-phenylamino-5,6-dihydro-1,3-thiazin-4-one and 2-(4-nitrophenylamino)-5,6-dihydro-1,3-thiazin-4-one and additionally discloses 2-hexylamino-5,6-dihydro-1,3-thiazin-4-one. Moreover, it indicates that 2-phenylamino-5,6-dihydro-1,3-thiazin-4-one exhibits fungicidal activity against Verticillium dahliae.

According to the invention there is therefore provided a fungicidal composition which comprises a carrier together with as active ingredient, a thiazinone compound of the general formula I:

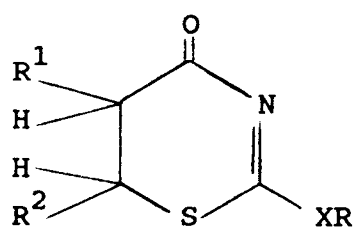

I

wherein X represents an oxygen or sulphur atom or the group NH; R represents an alkyl or alkenyl group of up to 6 carbon atoms; a pyridyl, pyrimidinyl, pyrazinyl, quinolyl, indolyl, benzimidazolyl, benzoxazolyl or coumarinyl group optionally substituted by a halogen atom, a nitro group or an alkyl group of up to 6 carbon atoms; a naphthyl or phenyl group optionally substituted by one or more substituents selected from halogen atoms, carboxyl, cyano, hydroxyl, amino and nitro groups, alkyl and haloalkyl groups of up to 6 carbon atoms, cycloalkyl groups of 3 to 8 carbon atoms, alkanoyl groups of up to 6 carbon atoms, alkoxy and alkylthio groups of up to 6 carbon atoms, alkylsulphinyl and alkylsulphonyl groups of up to 6 carbon atoms, alkoxycarbonylalkoxy groups of up to 8 carbon atoms, carbamoyl and alkylamido groups of up to 6 carbon atoms, imidazolyl groups, and phenyl and phenoxy groups optionally substituted by one or more halogen atoms or haloalkyl groups of up to 6 carbon atoms, or vicinal disubstituted by a thiadiazole ring; and $R^1$ and $R^2$ each represent a hydrogen atom or together represent a single carbon-carbon bond; with the proviso that, when X is NH and $R^1$ and $R^2$ both represent a hydrogen atom, then R is not a phenyl group.

Preferably X represents an oxygen or sulphur atom. It is also preferred that, when $R^1$ and $R^2$ together represent a single carbon-carbon bond, R represents an alkyl group of 1 to 4 carbon atoms; an allyl group; a pyridyl, methylpyridyl, nitropyridyl, methylpyrimidinyl, pyrazinyl, quinolyl, indolyl, benzimidazolyl or benzoxazolyl group; a naphthyl group optionally substituted by a bromine atom; or a phenyl group optionally substituted by 1 to 3 substituents selected from fluorine, chlorine and bromine atoms, carboxyl, hydroxyl, amino, nitro and cyano groups, methyl, propyl, trifluoromethyl, cyclohexyl, formyl, acetyl, propionyl, methoxy, methylthio, methylsulphinyl, methylsulphonyl, methoxycarbonylethoxy, acetamido, imidazolyl or phenyl groups, and phenoxy groups substituted by a chlorine atom and a trifluoromethyl group, or vicinal disubstituted by a thiadiazole ring. Preferably, when $R^1$ and $R^2$ each represent a hydrogen atom, R represents a phenyl group substituted by 1 or 2 substituents selected from chlorine atoms, methyl groups and trifluoromethyl groups.

A method of making a fungicidal composition as defined above is also provided which comprises bringing a thiazinone compound of formula I into association with at least one carrier.

The invention further provides a thiazinone compound of formula I, wherein X, R, $R^1$ and $R^2$ are as previously defined, with the provisos that, when X is NH, then R is not a tert-butyl or 2-methoxyphenyl

group; when X is NH and $R^1$ and $R^2$ both represent a hydrogen atom, then R is not a hexyl, 2-methylphenyl, 4-methylphenyl, 4-chlorophenyl, 4-nitrophenyl or naphthyl group; when X is NH and $R^1$ and $R^2$ together represent a single carbon-carbon bond, then R is not a phenyl group; and, when X is 0, S or NH and $R^1$ and $R^2$ together represent a single carbon-carbon bond, then R is not a methyl group.

The invention also provides a process for the preparation of a thiazinone compound of formula I as defined in the preceding paragraph which comprises reacting a compound of the general formula II

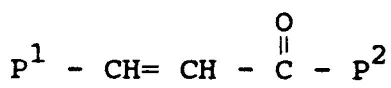

II

with a compound of the general formula III

RX - Q III

wherein X and R are as defined above and, when $R^1$ and $R^2$ in the resulting product of formula I each represent a hydrogen atom, $P^1$ represents a hydrogen atom, $P^2$ represents a halogen atom and Q represents a group

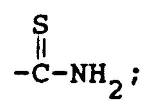

and, when $R^1$ and $R^2$ in the resulting product of formula I together represent a single carbon-carbon bond, $P^1$ and $P^2$ together represent a group

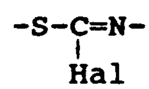

in which Hal represents a halogen atom and Q represents a hydrogen atom. The reaction is conveniently carried out in an inert organic solvent, such as acetone or dichloromethane at a temperature from room temperature to reflux temperature. Preferably it is carried out in the presence of a base, suitably an organic base such as a trialkylamine, triethylamine being most preferred.

The starting compound of formula II in which $P^1$ and $P^2$ together represent a group

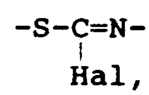

that is, the 2-halothiazinone compound, may conveniently be prepared from 3-thiocyanato-2-propenoic acid by reaction with phosphorus pentachloride followed by hydrogen chloride, suitably in an organic solvent such as ether.

The starting compound of formula III in which Q represents a group

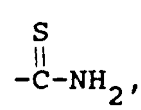

that is, the thiocarbamate compound, may be conveniently prepared by reacting a compound of formula RXCN in which R and X are as defined above with hydrogen sulphide, suitably in an organic solvent such as dimethyl formamide, ethanol or ether.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material

which is normally gaseous but which has been compressed to form a liquid, and any of the carriers normally used in formulating fungicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$-10% w of active ingredient. Granules are usually prepared to have a size between 10 and 100 BS mesh (1.676 - 0.152 mm), and may be manufactured by agglomeration or impregnation techniques. Generally, granules will contain $\frac{1}{2}$-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

Of particular interest in enhancing the duration of the protectant activity of the compounds of this invention is the use of a carrier which will provide a slow release of the fungicidal compounds into the environment of the plant which is to be protected. Such slow-release formulations could, for example, be inserted in the soil adjacent to the roots of a vine plant, or could include an adhesive component enabling them to be applied directly to the stem of a vine plant.

The invention still further provides the use as fungicide of a thiazinone compound of the general formula I as defined above, and a method for combating fungus at a locus, which comprises treating the locus, which may be for example be plants subject to or subjected to fungal attack, seeds of such plants or the medium in which such plants are growing or are to be grown, with such a compound.

The present invention is of wide applicability in the protection of crop plants against fungal attack. Typical crops which may be protected include vines, grain crops such as wheat and barley, rice, beans and apples. The duration of protection is normally dependent on the individual compound selected, and also a variety of external factors, such as climate, whose impact is normally mitigated by the use of a suitable formulation.

The invention is illustrated in the following Examples.

Example 1

A) Preparation of 3-thiocyanato-2-propenoic acid

Propiolic acid (100ml, 1.6mol) was added dropwise to a stirred solution of concentrated sulphuric acid (97ml) in water (550ml). The temperature was kept below 0 °C using an ice/methanol bath. A solution of potassium thiocyanate (167g, 1.72mol) in water (100ml) was then added dropwise, and a cream precipitate appeared. The mixture was kept in a fridge overnight, then the solid filtered, washed with water and dried in a vacuum oven at 60 °C for 18 hours, to yield the desired product, m.pt. 148-150 °C.

B) Preparation of 2-chloro-1,3-thiazin-4-one

The thiocyanato acid obtained in A (30g, 0.24mol) was stirred in diethylether (Na/Pd alloy dried, 400ml), and phosphorus pentachloride (50g, 0.24mol) was added. The suspension was stirred in an ice/methanol bath for 1½ hours after which time a colourless solution had formed. Dry hydrogen chloride gas was bubbled through the solution for 2 hours, the temperature being kept around -10 °C. The resulting white precipitate of 2-chloro-1,3-thiazin-4-one was filtered under nitrogen, dried in a vacuum oven, and promptly converted to the desired final product.

C) Preparation of 2-(4-nitrophenoxy)-1,3-thiazin-4-one

The 2-chloro-1,3-thiazin-4-one (17g, 0.115mol) obtained in B was stirred as a suspension in dichloromethane, and a solution prepared by adding triethylamine (16ml, 0.115mol) to 4-nitrophenol (14.2g, 0.115mol) in dichloromethane was added dropwise. The mixture was stirred for 18 hours, then the white precipitate was filtered, washed with water and dichloromethane, and dried in a vacuum oven to yield the desired final product, m.pt. 225-227 °C.

| Analysis | | | |
|---|---|---|---|
| Calculated:<br>Found: | C 48.0;<br>C 47.8; | H 2.4;<br>H 2.5; | N 11.2%<br>N 11.4% |

Example 2

Preparation of 2-(3-trifluoromethylphenoxy)-1,3-thiazin-4-one

2-Chloro-1,3-thiazin-4-one, prepared by the procedure described in Examples 1A and 1B, (3g, 0.02mol) was stirred as a suspension in dichloromethane, and a solution prepared by adding triethylamine (2.8ml, 0.02mol) to 3-trifluoromethylphenol (3.24g, 0.02mol) dropwise. The mixture was stirred for 18 hours and a clear solution resulted. The crude reaction product was purified by flash chromatography using 2% methanol in dichloromethane as the eluant, yielding the desired product as a white solid, m.pt. 123 °C

| Analysis | | | |
|---|---|---|---|
| Calculated:<br>Found: | C 48.3;<br>C 48.4; | H 2.2;<br>H 2.4; | N 5.1%<br>N 5.3% |

Example 3

Preparation of 2-(4-methylanilino)-1,3-thiazin-4-one

2-chloro-1,3-thiazin-4-one, prepared by the procedure described in Examples 1A and 1B, (2.0g, 0.0135m) was stirred as a suspension in dichloromethane and a solution prepared by adding triethylamine (1.36g, 0.0135m) to p-toluidine (1.45g, 0.0135m) in dichloromethane was added dropwise. The resulting mixture was stirred for 18 hours at room temperature, and then purified by flash chromatography using 5:95 metanol:dichloromethane, to yield the desired product as a white solid, m.pt. 176-178 ° C.

| Analysis | | | |
|---|---|---|---|
| Calculated:<br>Found: | C 60.5;<br>C 60.2; | H 4.6;<br>H 4.5; | N 12.8%<br>N 12.8% |

Examples 4-85

Following procedures similar to those described in Examples 1-3 above, further 2-substituted-1,3 thiazinones were prepared, whose physical characteristics and analyses are given in Table I below. In this Table, the compounds are identified by reference to formula I and, in all cases, $R^1$ and $R^2$ together represent a carbon-carbon bond.

Table I

| Example No. | X | R | M.Pt. °C | C Calc. | C Found | H Calc. | H Found | N Calc. | N Found |
|---|---|---|---|---|---|---|---|---|---|
| 4 | O | Phenyl | 92-93 | 58.5 | 57.4 | 3.4 | 3.6 | 6.8 | 6.7 |
| 5 | O | 4-Cl Phenyl | 184-186 | 50.1 | 49.9 | 2.5 | 2.7 | 5.8 | 6.0 |
| 6 | O | 3-Cl Phenyl | 131-133 | 50.1 | 50.3 | 2.5 | 2.7 | 5.8 | 5.9 |
| 7 | O | 2-F Phenyl | 131-133 | 53.8 | 53.8 | 2.7 | 3.0 | 6.3 | 6.4 |
| 8 | O | 4-F Phenyl | 183 | 53.8 | 53.8 | 2.7 | 2.8 | 6.3 | 6.6 |
| 9 | O | 3-CN Phenyl | 190 | 57.4 | 57.6 | 2.6 | 2.8 | 12.2 | 12.2 |
| 10 | O | 4-CN Phenyl | 246 | 57.4 | 57.1 | 2.6 | 2.6 | 12.2 | 12.2 |
| 11 | O | 4-Tolyl | 125-127 | 60.3 | 61.4 | 4.1 | 4.4 | 6.4 | 6.1 |
| 12 | O | 2-$NO_2$ Phenyl | 186 | 48.0 | 48.0 | 2.4 | 2.6 | 11.2 | 11.3 |
| 13 | O | 3-$NO_2$ Phenyl | 201 | 48.0 | 48.1 | 2.4 | 2.5 | 11.2 | 11.3 |
| 14 | O | 2-$NO_2$, 5-Acetyl Phenyl | 184-186 | 49.3 | 49.6 | 2.7 | 2.8 | 9.6 | 9.6 |
| 15 | O | 2-$NO_2$, 3-Acetyl Phenyl | 187-189 | 49.3 | 48.9 | 2.7 | 2.7 | 9.6 | 9.6 |
| 16 | O | 4-$NO_2$, 3-Acetyl Phenyl | 175-176 | 49.3 | 49.1 | 2.7 | 2.7 | 9.6 | 9.7 |
| 17 | O | 2-$NO_2$, 5-Cl Phenyl | 192-193 | 42.1 | 42.2 | 1.8 | 1.7 | 9.8 | 9.8 |

TABLE I (continued)

| Example No. | X | R | M.Pt.°C | Analysis | | | | | |
|---|---|---|---|---|---|---|---|---|---|
| | | | | C | | H | | N | |
| | | | | Calc. | Found | Calc. | Found | Calc. | Found |
| 18 | O | 4-$NO_2$, 2,6-$Cl_2$ Phenyl | 200-201 | 37.6 | 37.8 | 1.3 | 1.6 | 8.8 | 8.9 |
| 19 | O | 4-$NO_2$, 2,6-$Br_2$ Phenyl | 220-221 | 29.4 | 29.7 | 1.0 | 1.2 | 6.9 | 6.9 |
| 20 | O | 4-Acetyl Phenyl | 200-201 | 58.3 | 58.3 | 3.6 | 3.7 | 5.7 | 5.7 |
| 21 | O | 4-Propionyl Phenyl | 156-157 | 60.0 | 60.1 | 4.2 | 4.0 | 5.4 | 5.4 |
| 22 | O | 2-Methoxy Phenyl | 127-129 | 56.2 | 56.9 | 3.8 | 3.9 | 6.0 | 5.9 |
| 23 | O | 3-Methoxy Phenyl | 93-95 | 56.2 | 57.7 | 3.8 | 4.0 | 6.0 | 5.9 |
| 24 | O | 4-Methoxy Phenyl | 136-138 | 56.2 | 57.7 | 3.8 | 4.0 | 6.0 | 5.9 |
| 25 | O | 4-Methylthio Phenyl | 178 | 52.6 | 52.6 | 3.6 | 3.6 | 5.6 | 5.9 |
| 26 | O | 2-$CF_3$ Phenyl | 184-185 | 48.4 | 48.8 | 2.2 | 2.3 | 5.1 | 5.1 |
| 27 | O | 4-$CF_3$ Phenyl | 143 | 48.4 | 48.9 | 2.2 | 2.4 | 5.1 | 5.4 |
| 28 | O | 4-$CF_3$ 2-Cl Phenyl | 167-168 | 43.0 | 43.8 | 1.6 | 1.7 | 4.6 | 4.7 |
| 29 | O | 4-Carboxyl Phenyl | 208 (dec) | 53.0 | 53.9 | 2.8 | 3.1 | 5.6 | 5.8 |
| 30 | O | 4-$OCH(CH_3)$ $COOCH_3$ Phenyl | 124-126 | 54.7 | 54.8 | 4.2 | 4.5 | 4.6 | 4.5 |
| 31 | O | 4-Cyclohexyl Phenyl | 186-187 | 66.9 | 66.9 | 5.9 | 5.9 | 4.9 | 5.1 |

TABLE I (continued)

| Example No. | X | R | M.Pt.°C | Analysis C Calc. | C Found | H Calc. | H Found | N Calc. | N Found |
|---|---|---|---|---|---|---|---|---|---|
| 32 | 0 | 2-Biphenylyl | 156-157 | 68.3 | 68.3 | 3.9 | 4.0 | 5.0 | 5.4 |
| 33 | 0 | 4-$SOCH_3$ Phenyl | 188 | 49.4 | 49.3 | 3.5 | 3.5 | 5.5 | 5.5 |
| 34 | 0 | 4-$SO_2$ $CH_3$ Phenyl | 186 | 46.6 | 46.6 | 3.3 | 3.3 | 5.1 | 5.1 |
| 35 | 0 | 1-Naphthyl | 166 | 65.8 | 65.6 | 3.5 | 3.5 | 5.5 | 5.5 |
| 36 | 0 | 2-Naphthyl | 206-207 | 65.8 | 65.3 | 3.5 | 3.6 | 5.5 | 5.6 |
| 37 | 0 | 2-Pyridyl | 172 | 52.4 | 51.9 | 2.9 | 3.4 | 13.6 | 13.9 |
| 38 | 0 | 5-$NO_2$, 2-Pyridyl | 234(dec) | 43.0 | 42.8 | 2.0 | 2.0 | 16.7 | 16.7 |
| 39 | 0 | 3-Pyridyl (N-oxide) | 173 | 48.6 | 48.5 | 2.7 | 2.7 | 12.6 | 12.6 |
| 40 | 0 | 8-Quinolyl | 217 | 60.9 | 60.7 | 3.1 | 3.0 | 10.9 | 10.8 |
| 41 | 0 | 3,4-thiadiazolo Phenyl | 176-177 | 45.6 | 43.9 | 1.9 | 2.0 | 16.0 | 14.9 |
| 42 | S | $CH_3$ | 151-153 | 37.7 | 37.6 | 3.1 | 3.2 | 8.8 | 8.8 |
| 43 | S | $C_3H_7$ | 187 | 44.9 | 44.8 | 4.8 | 4.9 | 7.5 | 7.6 |
| 44 | S | Phenyl | 159-160 | 54.3 | 54.0 | 3.2 | 3.2 | 6.3 | 6.4 |
| 45 | S | 4-F Phenyl | 178 | 50.2 | 50.1 | 2.5 | 2.7 | 5.9 | 6.0 |

TABLE I (continued)

| Example No. | X | R | M.Pt. °C | Analysis C Calc. | C Found | H Calc. | H Found | N Calc. | N Found |
|---|---|---|---|---|---|---|---|---|---|
| 46 | S | 4-Cl Phenyl | 181 | 47.0 | 46.6 | 2.4 | 2.5 | 5.5 | 5.7 |
| 47 | S | 2,4,5-$Cl_3$ Phenyl | 178-179 | 37.0 | 37.1 | 1.2 | 1.4 | 4.3 | 4.4 |
| 48 | S | 4-Tolyl | 187 | 56.2 | 56.0 | 3.8 | 3.9 | 6.0 | 6.7 |
| 49 | S | 4-$NO_2$ Phenyl | 170-171 | 45.1 | 45.6 | 2.2 | 2.2 | 10.5 | 10.5 |
| 50 | S | 2-Methoxy Phenyl | 132 | 52.6 | 52.4 | 3.6 | 3.5 | 5.6 | 6.0 |
| 51 | S | 4-Pyridyl | 128 | 48.6 | 48.5 | 2.7 | 2.8 | 12.6 | 12.3 |
| 52 | S | 2-Pyridyl | 123-124 | 48.6 | 48.6 | 2.7 | 2.8 | 12.6 | 12.3 |
| 53 | NH | $CH_3$ | 185-187 | 42.3 | 42.5 | 4.2 | 4.3 | 19.7 | 19.4 |
| 54 | NH | $C_3H_7$ | 156-158 | 49.4 | 49.2 | 5.6 | 6.0 | 16.5 | 16.4 |
| 55 | NH | Allyl | 143-145 | 50.0 | 49.8 | 4.8 | 4.5 | 16.7 | 16.6 |
| 56 | NH | 2-F Phenyl | 133-135 | 54.1 | 53.7 | 3.2 | 3.0 | 12.6 | 12.2 |
| 57 | NH | 3-Cl Phenyl | 182-184 | 50.3 | 50.2 | 2.9 | 3.1 | 11.7 | 11.6 |
| 58 | NH | 4-Cl Phenyl | 175-177 | 50.3 | 50.2 | 2.9 | 3.0 | 11.7 | 11.6 |
| 59 | NH | 4-$C_3H_7$ Phenyl | 124-128 | 63.4 | 63.2 | 5.7 | 5.5 | 11.4 | 11.5 |

TABLE I (continued)

| Example No. | X | R | M.Pt.°C | Analysis C Calc. | C Found | H Calc. | H Found | N Calc. | N Found |
|---|---|---|---|---|---|---|---|---|---|
| 60 | NH | 3-$CF_3$ Phenyl | 167-169 | 48.5 | 47.5 | 2.6 | 2.5 | 10.3 | 10.0 |
| 61 | NH | 3-$CF_3$, 4-Cl Phenyl | 185-187 | 43.1 | 43.0 | 2.0 | 2.0 | 9.1 | 9.3 |
| 62 | NH | 4-(2-Cl, 4-$CF_3$ phenoxy) Phenyl | 194-196 | 51.2 | 51.2 | 2.5 | 2.6 | 7.0 | 7.2 |
| 63 | NH | 4,6-$(CH_3)_2$ Pyrimidin-2-yl | 198-200 | 51.3 | 50.6 | 4.3 | 4.3 | 23.9 | 22.3 |
| 64 | O | 3-F Phenyl | 138-139 | 53.8 | 54.2 | 2.7 | 2.7 | 6.3 | 6.4 |
| 65 | O | 2-Cl Phenyl | 158-159 | 50.2 | 50.2 | 2.7 | 2.6 | 6.3 | 6.4 |
| 66 | O | 2,4-$Cl_2$ Phenyl | 184-185 | 43.9 | 43.6 | 1.8 | 2.0 | 5.1 | 5.4 |
| 67 | O | 2,6-$Cl_2$ Phenyl | 181-182 | 43.9 | 43.8 | 1.8 | 1.9 | 5.1 | 5.3 |
| 68 | O | 4-Br Phenyl | 175-176 | 42.3 | 42.3 | 2.1 | 2.2 | 4.9 | 5.0 |
| 69 | O | 2-$CH_3$,4-Cl Phenyl | 181-182 | 52.2 | 52.0 | 3.2 | 3.3 | 5.5 | 5.7 |
| 70 | O | 2,6-$Cl_2$,4-$NH_2$ Phenyl | 212-213 | 41.7 | 41.3 | 2.1 | 2.5 | 9.7 | 9.7 |
| 71 | O | 2,4-$(NO_2)_2$ Phenyl | 203-204 | 40.7 | 40.7 | 1.7 | 1.8 | 14.2 | 14.2 |
| 72 | O | 3-$CH_3$,4-$NO_2$ Phenyl | 194-195 | 50.0 | 50.2 | 3.0 | 3.2 | 10.6 | 10.6 |
| 73 | O | 3-Formyl Phenyl | 130 | 56.6 | 56.6 | 3.0 | 3.1 | 6.0 | 6.2 |

TABLE I (continued)

| Example No. | X | R | M.Pt.°C | Analysis C Calc. | C Found | H Calc. | H Found | N Calc. | N Found |
|---|---|---|---|---|---|---|---|---|---|
| 74 | O | 4-Formyl Phenyl | 167-168 | 56.6 | 56.7 | 3.0 | 3.1 | 6.0 | 6.0 |
| 75 | O | 4-Acetamido Phenyl | 168-169 | 55.0 | 54.7 | 3.8 | 4.0 | 10.7 | 10.9 |
| 76 | O | 4-(1-Imidazolyl)Phenyl | >300 | 57.6 | 57.2 | 3.3 | 3.3 | 15.5 | 15.4 |
| 77 | O | 6-Br,2-Naphthyl | 199-200 | 50.3 | 49.9 | 2.4 | 2.7 | 4.2 | 4.5 |
| 78 | O | 6-$CH_3$,3-Pyridyl | 94-95 | 54.5 | 54.2 | 3.6 | 3.8 | 12.7 | 12.4 |
| 79 | O | 2-Pyrazinyl | 150-151 | 45.7 | 45.8 | 2.4 | 2.7 | 20.0 | 19.8 |
| 80 | O | 5-Indolyl | 162 | 59.0 | 58.8 | 3.3 | 3.5 | 11.5 | 11.2 |
| 81 | S | 3-Cl Phenyl | 167-168 | 47.1 | 46.8 | 2.4 | 2.4 | 5.5 | 5.6 |
| 82 | S | 3-$CF_3$ Phenyl | 180-181 | 45.7 | 45.5 | 2.1 | 2.1 | 4.8 | 5.0 |
| 83 | S | 4-Acetamido Phenyl | 183-184 | 51.8 | 51.4 | 3.4 | 3.8 | 10.1 | 10.1 |
| 84 | S | 2-Benzimidazolyl | 159-160 | 50.6 | 50.7 | 2.7 | 2.8 | 16.1 | 16.0 |
| 85 | S | 2-Benzoxazolyl | 237 | 50.4 | 50.1 | 2.3 | 2.3 | 10.7 | 10.7 |

Example 86

A) Preparation of O-(4-chloro-2-methyl)phenyl thiocarbamate

Hydrogen sulphide gas was passed through a solution of 4-chloro-2-methylphenyl cyanate (4g 0.024mol)

in dry dimethyl formamide (30ml) for 2 hours. The mixture was stirred overnight, then water (30ml) was added. The solution was extracted with dichloromethane (60ml), and the organic layer was dried over magnesium sulphate. Concentration by rotary evaporation gave an orange oil. This was purified by flash chromatography, using 25:75 ethylacetate: petrol, to yield the desired product as a white solid, m.pt. 150 °C.

| Analysis | | | |
|---|---|---|---|
| Calculated:<br>Found: | C 47.6;<br>C 47.5; | H 4.0;<br>H 4.0; | N 6.9%<br>N 6.5% |

B) Preparation of 2-(4-chloro-2-methyl)phenoxy-5,6-dihydrothiazinone

Triethylamine (1ml. 0.007 mol) was added to a solution of the O-(4-chloro-2-methyl)phenyl thiocarbamate obtained in A (1.5g, 0.007 mol) and acryloyl chloride (0.62 ml, 0.007 mol) in acetone (20ml). The resulting solution was refluxed for 24 hours. After cooling the acetone was removed by rotary evaporation and the residue taken up in dichloromethane and then washed with water. The organic layer was dried over magnesium sulphate and then concentrated by rotary evaporation. The residue was purified by flash chromatography using 25:75 ethyl acetate: petrol to yield the desired final product, m.pt. 128 °C.

| Analysis | | | |
|---|---|---|---|
| Calculated:<br>Found: | C 51.7;<br>C 51.4; | H 3.9;<br>H 4.0; | N 5.5%<br>N 5.8% |

Example 87

2-(4-trifluoromethyl)phenoxy-5,6-dihydrothiazinone was prepared by procedures similar to those described in Example 86 above.

| Analysis | | | |
|---|---|---|---|
| Calculated:<br>Found: | C 48.0;<br>C 47.9; | H 2.9;<br>H 3.2; | N 5.1%<br>N 5.9% |

Example 88

The fungicidal activity of compounds of the invention was determined by means of the following tests.

a) Antisporulant activity against vine downy mildew (Plasmopara viticola; P.v.a)

The test is a direct antisporulant one using a foliar spray. The lower surfaces of leaves of whole vine plants (cv Cabernet Sauvignon) are inoculated by spraying with an aqueous suspension containing $10^4$ zoosporangia/ml 2 days prior to treatment with the test compound. The inoculated plants are kept for 24 hours in a high humidity compartment, and then 24 hours at glasshouse ambient temperature and humidity. Infected leaves are sprayed on their lower surfaces with a solution of active material in 1:1 water/acetone containing 0.04% "TWEEN" 20 (Trade Mark; a polyoxyethylene sorbitan ester surfactant). The spraying is carried out with a moving track sprayer giving an application rate of 1kg/ha. After spraying, the plants are returned to normal glasshouse conditions for 96 hours and are then transferred to the high humidity compartment for 24 hours to induce sporulation, prior to assessment. Assessment is based on the percentage of the leaf area covered by sporulation compared with that on control leaves.

c) Direct protectant activity against vine downy mildew (Plasmopara viticola; P.v.p)

The test is a direct protectant one using a foliar spray. The lower surfaces of leaves of whole vine plants (cv Cabernet Sauvignon) are sprayed with the test compound at a dosage of 1 kilogram of active material per hectare using a track sprayer as described under (a), and after a subsequent 24 hours under normal glasshouse conditions the lower surfaces of the leaves are inoculated by spraying with an aqueous solution containing $10^4$ zoosporangia/ml. The inoculated plants are kept for 24 hours in a high humidity compartment, 5 days under normal glasshouse conditions and then returned for a further 24

hours to high humidity. Assessment is based on the percentage of leaf area covered by sporulation compared with that on control leaves.

d) Direct protectant activity against vine grey mould (Botrytis cinerea; Bcp)

The test is a direct protectant one using a foliar spray. The lower surfaces of detached vine leaves (cv Cabernet Sauvignon) are sprayed with the test compound at a dosage of 1kg/ha using a track sprayer as in (a). 24 Hours after spraying the leaves are inoculated with droplets of aqueous suspension containing $10^5$ conidia/ml. After a further 5 days in high humidity the percentage of leaf area covered by disease is assessed.

e) Activity against wheat leafspot (Leptosphaeria nodorum;

The test is a direct therapeutic one, using a foliar spray. Leaves of wheat plants (cv Mardler), at the single leaf stage, are inoculated by spraying with an aqueous suspension containing $1x10^6$ spores/ml. The inoculated plants are kept for 24 hours in a high humidity compartment prior to treatment. The plants are sprayed with a solution of the test compound at a dosage of 1 kilogram of active material per hectare using a track sprayer as described under (a). After drying, the plants are kept for 6-8 days at 20-25°C and moderate humidity, followed by assessment. Assessment is based on the density of lesions per leaf compared with that on leaves of control plants.

f) Activity against barley powdery mildew (Erysiphe graminis f.sp. hordei; Eg)

The test is a direct therapeutic one, using a foliar spray. Leaves of barley seedlings, (cv. Golden Promise) are inoculated by dusting with mildew conidia one day prior to treatment with the test compound. The inoculated plants are kept overnight at glasshouse ambient temperature and humidity prior to treatment. The plants are sprayed with the test compound at a dosage of 1 kilogram of active material per hectare using a track sprayer as described under (a). After drying, plants are returned to a compartment at 20-25°C and moderate humidity for up to 7 days, followed by assessment. Assessment is based on the percentage of leaf area covered by sporulation compared with that on leaves of control plants.

g) Activity against apple powdery mildew (Podosphaera leucotricha; Pl)

The test is a direct therapeutic one using a foliar spray. The upper surfaces of leaves of apple seedlings are inoculated by spraying with an aqueous suspension containing $10^5$ conidia/ml 2 days prior to treatment with the test compounds. The inoculated plants are immediately dried and kept at glasshouse ambient temperatures and humidity prior to treatment. The plants are sprayed with the test compound at a dosage of 1 kilogram of active material per hectare using a track sprayer as described under (a). After drying the plants are returned to a compartment at 20-25°C and moderate humidity for up to 9 days, followed by assessment. Assessment is based on the percentage of the leaf area covered by sporulation compared with that on leaves of control plants.

h) Activity against broad bean rust (Uromyces fabae Uf)

The test is a therapeutic one using a foliar spray. Pots containing 1 plant per pot are inoculated by spraying an aqueous suspension, containing $5x10^4$ spores/ml plus a little "Tween 20" (Trade Mark), onto the upper surface of each leaf 20-24 hours before treatment with test compound. The inoculated plants were kept overnight in a high humidity compartment, dried at glasshouse ambient temperature and then sprayed, on the leaf upper surface, with the test compound at a dosage of 1 kilogram of active material per hectare using a track sprayer as described under (a). After treatment the plants were kept at glasshouse temperature and assessment made 11-14 days after treatment. Symptoms are assessed on the relative density of sporulating pustules per plant compared with that on control plants.

i) Activity against rice leaf blast (Pyricularia oryzae Po)

The test is a direct therapeutic one using a foliar spray. The leaves of rice seedlings (about 30 seedlings per pot) are sprayed with an aqueous suspension containing $10^5$ spores/ml 20-24 hours prior to treatment with the test compound. The inoculated plants are kept overnight in high humidity and then allowed to dry before spraying with the test compound at a dosage of 1 kilogram of active material per hectare using a track sprayer as described under (a). After treatment the plants are kept in a rice compartment at 25-30°C and high humidity. Assessments are made 4-5 days after treatment and are based on the density of necrotic lesions per leaf when compared with control plants.

j) Activity against tomato early blight (Alternaria solani; As)

This test measures the contact prophylactic activity of test compounds applied as a foliar spray.

Tomato seedlings (cv Outdoor Girl) are grown to the stage at which the second true leaf is expanded. The plants are treated using a track sprayer as described under (a). Test compounds are applied as solutions or suspensions in a mixture of acetone and water (50:50v/v) containing 0.04% surfactant ("TWEEN 20" - Trademark).

One day after treatment the seedlings are inoculated by spraying the leaf upper surfaces with a

suspension of A. solani conidia containing $10^4$ spores/ml. For 3 days after inoculation plants are kept moist in a glasshouse compartment at or near 100% RH and 21°C. Thereafter plants are kept under humid, but not saturated, conditions.

Disease is assessed 7 days after inoculation, based on the density and spread of lesions.

k) Activity against wheat eyespot in-vitro (Pseudoceroosporella herpotrichoides; PhI)

This test measures the in vitro activity of compounds against the fungus causing wheat eyespot.

The Test Compound is dissolved or suspended in acetone and is added to molten half strength Potato Dextrose Agar to give a final concentration of 100ppm compound and 3.5% acetone. After agar has set, plates are inoculated with 6mm diameter plugs of agar/mycelium taken from a 14 day old culture of P. herpotrichoides.

Plates are incubated at 20°C for 12 days and radial growth from the inoculation plug is measured.

l) Activity against Fusarium in-vitro (Fusarium species; FsI)

This test measures the in vitro activity of compounds against a species of Fusarium that causes stem and root rots.

Compound is dissolved or suspended in acetone and added to molten half strength Potato Dextrose Agar to give a final concentration of 100ppm compound and 3.5% acetone. After agar has set, plates are inoculated with 6mm diameter plugs of agar and mycelium taken from a 7 day old culture of Fusarium sp..

Plates are incubated at 20°C for 5 days and radial growth from the plug is measured.

The extent of disease control in all the above tests is expressed as a rating compared with either an untreated control or a diluent-spray-control, according to the criteria:-

0 = less than 50% disease control

1 = about 50-80% disease control

2 = greater than 80% disease control

The results of these tests are set out in Table II below.

Table II

| Example No. | Fungicidal Evaluation |
|---|---|
| 1 | Pvp 2; Bcp 1; As 2 |
| 2 | Ph 2 |
| 3 | Pvp 1; Uf 1 |
| 4 | Pl 2; Fs 2 |
| 5 | Pva 1; Pvp 2; As 1 |
| 6 | Pva 1; Pvp 2; Bcp 1; Po 1; As 2 |
| 7 | Pva 1; Pvp 2; Bcp 1; Ln 1 |
| 8 | Pvp 1; Ln 1; Ph 1 |
| 9 | Pvp 1; Ph 2 |
| 10 | Pvp 1; As 1; Ph 1 |
| 11 | Pvp 2 |
| 12 | As 1; Ph 2 |
| 13 | Bcp 1; Ph 1, Fs 1 |
| 14 | Pvp 1; Pl 1; As 2; Fs 1; Ph 2 |
| 15 | Pvp 1; As 2; Ph 2 |
| 16 | Pvp 2 |
| 17 | Pvp 2; Ph 2 |
| 18 | Fs 2; Po 1 |
| 19 | Fs 1 |
| 20 | Ph 1; Fs 1 |
| 21 | Pvp 1; As 2; Ph 2; Fs 2 |
| 22 | Pvp 1; Ln 2; Eg 1; Pl 2 |
| 23 | Eg 1; Pl 1; Ph 1 |
| 24 | Ph 1 |
| 25 | Pvp 1; Bcp 1; Ln 1; Ph 2 |
| 26 | Pvp 2; As 1; Pl 1; Ph 1; Fs 1 |
| 27 | Pvp 2; Eg 1; Ph 2 |
| 28 | Pvp 2; As 2; Ph 2; Fs 2 |
| 29 | Pvp 1 |
| 30 | Pvp 2; Eg 1 |

Table II (continued).

| Example No. | Fungicidal Evaluation |
|---|---|
| 32 | Pvp 2; As 1; Pl 1; Ph 1; Fs 2 |
| 33 | Pvp 1; Pl 1 |
| 34 | Pl 1; Fs 1 |
| 35 | Pvp 1; Ph 1; Fs 2 |
| 36 | Pvp 1; Bcp 1; Uf 1; Ph 1; Fs 1 |
| 37 | As 1; Ph 2 |
| 38 | Pvp 1 |
| 39 | Pvp 1 |
| 40 | Pvp 1; Bcp 2; Ph 2 |
| 41 | Pvp 2; As 2; Ph 2; Fs 2 |
| 42 | Pvp 2; Ln 1; Po 1; Fs 1 |
| 43 | Po 1 |
| 44 | Pvp 2; Bcp 2; As 1; Ph 2; Fs 2 |
| 45 | Fs 2; Ph 2; Pvp 1; Bcp 1 |
| 46 | Pvp 1; Ln 2; Eg 1; Pl 2 |
| 47 | Pvp 1 |
| 48 | Pvp 1; Bcp 1; Eg 2; Ph 2 |
| 49 | Pvp 1; Bcp 2; Ph 2 |
| 50 | Pvp 1; As 1; Ph 2 |
| 51 | Pvp 1; Bcp 2; Ph 2; Fs 1 |
| 52 | Eg 1; Ph 2; Fs 1 |
| 54 | Pvp 1; Po 1 |
| 55 | Bcp 1 |
| 56 | Pvp 1; As 1 |
| 57 | Pvp 1; As 2 |
| 58 | Pvp 1; Bcp 1 |
| 59 | Pvp 1; As 1 |
| 60 | Fs 2 |
| 61 | Po 1; Fs 1 |
| 64 | Pvp 2; Ph 1; Fs 1 |

Table II (continued)

| Example No. | Fungicidal Evaluation |
|---|---|
| 65 | Pvp 2; Ph 1; Fs 1 |
| 66 | Pvp 2; As 2; Ph 1; Fs 1 |
| 67 | Pvp 2; As 1; Ph 1; Fs 1 |
| 68 | Pvp 2; Bcp 1; Ph 1; Fs 2 |
| 69 | Pvp 2; As 1; Ph 1; Fs 1 |
| 70 | Pvp 2 |
| 71 | Pvp 2; As 1 |
| 72 | Pvp 2; As 2; Ph 2 |
| 73 | Pvp 1; Ph 1; Fs 1 |
| 74 | Pvp 1; Ph 1; Fs 1 |
| 75 | Ln 1 |
| 76 | Pvp 2 |
| 77 | As 1 |
| 78 | Pvp 1; Fs 1 |
| 79 | Pvp 2 |
| 80 | Pvp 2 |
| 81 | Pvp 2; Ph 2; Fs 2 |
| 82 | Pvp 2; As 1 |
| 83 | Pvp 1 |
| 84 | Pvp 2; As 1; Ph 1; Fs 1 |
| 85 | Pvp 2; Ph 2; Fs 1 |
| 86 | Pvp 1; Ph 2; Fs 2 |
| 87 | Pvp 1; Ph 2; Fs 1 |

## Claims

1. A fungicidal composition which comprises a carrier together with, as active ingredient, a thiazinone compound of the general formula I:-

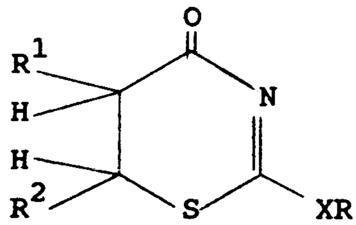

I

wherein X represents an oxygen or sulphur atom or the group NH, R represents an alkyl or alkenyl group of up to 6 carbon atoms; a pyridyl, pyrimidinyl, pyrazinyl, quinolyl, indolyl, benzimidazolyl, benzoxazolyl or coumarinyl group optionally substituted by a halogen atom, a nitro group or an alkyl group of up to 6 carbon atoms; a naphthyl or phenyl group optionally substituted by one or more substituents selected from halogen atoms, carboxyl, cyano, hydroxyl, amino and nitro groups, alkyl and haloalkyl groups of up to 6 carbon atoms, cycloalkyl groups of 3 to 8 carbon atoms, alkanoyl groups of up to 6 carbon atoms, alkoxy and alkylthio groups of up to 6 carbon atoms, alkylsulphinyl and alkylsulphonyl groups of up to 6 carbon atoms, alkoxycarbonylalkoxy groups of up to 8 carbon atoms, carbamoyl and alkylamido groups of up to 6 carbon atoms, imidazolyl groups, and phenyl and phenoxy groups optionally substituted by one or more halogen atoms or haloalkyl groups of up to 6 carbon atoms, or vicinal disubstituted by a thiadiazole ring; and $R^1$ and $R^2$ each represent a hydrogen atom or together represent a single carbon-carbon bond; with the proviso that, when X is NH and $R^1$ and $R^2$ both represent a hydrogen atom, then R is not a phenyl group.

**2.** A composition as claimed in claim 1 wherein X is an oxygen or sulphur atom.

**3.** A composition as claimed in claim 1 or claim 2 wherein R represents an alkyl group of 1 to 4 carbon atoms; an allyl group; a pyridyl, methylpyridyl, nitropyridyl, methylpyrimidinyl, pyrazinyl, quinolyl, indolyl, benzimidazolyl or benzoxazolyl group; a naphthyl group optionally substituted by a bromine atom; or a phenyl group optionally substituted by 1 to 3 substituents selected from fluorine, chlorine and bromine atoms, carboxyl, hydroxyl, amino, nitro and cyano groups, methyl, propyl, trifluoromethyl, cyclohexyl, formyl, acetyl, propionyl, methoxy, methylthio, methylsulphinyl, methylsulphonyl, methoxycarbonylethoxy, acetamido, imidazolyl and phenyl groups and phenoxy groups substituted by a chlorine atom and a trifluoromethyl group, or vicinal disubstituted by a thiadiazole ring; and $R^1$ and $R^2$ together represent a single carbon-carbon bond.

**4.** A composition as claimed in claim 1 or claim 2 wherein R represents a phenyl group substituted by 1 or 2 substituents selected from chlorine atoms, methyl groups and trifluoromethyl groups and $R^1$ and $R^2$ each represent a hydrogen atom.

**5.** A composition as claimed in any one of claims 1 to 4 which comprises at least two carriers, at least one of which is a surface-active agent.

**6.** A method of making a fungicidal composition as defined in any one of claims 1 to 4 which comprises bringing a thiazinone compound as defined in claim 1 into association with at least one carrier.

**7.** A thiazinone compound of the general formula I as shown in claim 1, wherein X, R, $R^1$ and $R^2$ are as defined in claim 1, with the provisos that, when X is NH, then R is not a tert-butyl or 2-methoxyphenyl group; when X is NH and $R^1$ and $R^2$ both represent a hydrogen atom, then R is not a hexyl, 2-methylphenyl, 4-methylphenyl, 4-chlorophenyl, 4-nitrophenyl or naphthyl group; when X is NH and $R^1$ and $R^2$ together represent a single carbon-carbon bond, then R is not a phenyl group; and, when X is 0, S or NH and $R^1$ and $R^2$ together represent a single carbon-carbon bond, then R is not a methyl group.

**8.** A process for the preparation of a thiazinone compound of the general formula I as defined in claim 7 which comprises reacting a compound of the general formula II

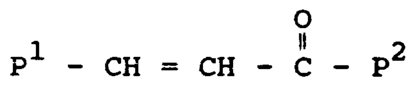 II

with a compound of the general formula III

RX - Q III

wherein X and R are as defined in claim 7 and, when $R^1$ and $R^2$ in the resulting product of formula I each represent a hydrogen atom, $P^1$ represents a hydrogen atom, $P^2$ represents a halogen atom and Q represents a group

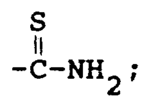

and, when $R^1$ and $R^2$ in the resulting product of formula I together represent a single carbon-carbon bond, $P^1$ and $P^2$ together represent a group

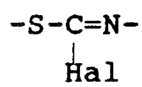

in which Hal represents a halogen atom and Q represents a hydrogen atom.

**9.** A process as claimed in claim 8 wherein the reaction is carried out in an inert organic solvent in the presence of a base.

**10.** A thiazinone compound as claimed in claim 7, whenever prepared by a process as claimed in claim 8 or claim 9.

**11.** A method of combating fungus at a locus, wherein the locus comprises plants subject to or subjected to fungal attack, seeds of such plants, or the medium in which the plants are growing or are to be grown, characterised by treating the locus with a fungicidally effective amount of a composition as claimed in any one of claims 1-5 or a compound as claimed in claim 7 or claim 10.

**12.** The use as a fungicide of a compound of formula I as defined in any one of claims 1-4, 7 and 10.

**Patentansprüche**

**1.** Fungizide Zusammensetzung, die einen Träger zusammen mit, als aktivem Bestandteil, einer Thiazinonverbindung der allgemeinen Formel I:

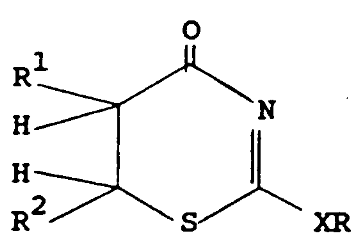 I

umfaßt, worin

X ein Sauerstoff- oder Schwefelatom oder die Gruppe NH bedeutet;

R eine Alkyl- oder Alkenylgruppe mit bis zu 6 Kohlenstoffatomen; eine Pyridyl-,

Pyrimidinyl-, Pyrazinyl-, Chinolyl-, Indolyl-, Benzimidazolyl-, Benz-oxazolyl- oder Cumarinylgruppe, gegebenenfalls substituiert durch ein Halogenatom, eine Nitrogruppe oder eine Alkylgruppe mit bis zu 6 Kohlenstoffatomen; eine Naphthyl- oder Phenylgruppe, gegebenenfalls substituiert durch einen oder mehrere Substituenten, ausgewählt unter Halogenatomen, Carboxyl-, Cyano-, Hydroxyl-, Amino- und Nitrogruppen, Alkyl- und Halogenalkylgruppen bis zu 6 Kohlenstoffatomen, Cycloalkylgruppen mit 3 bis 8 Kohlenstoffatomen, Alkanoylgruppen mit bis zu 6 Kohlenstoffatomen, Alkoxy- und Alkylthiogruppen mit bis zu 6 Kohlenstoffatomen, Alkylsulfinyl- und Alkylsulfonylgruppen mit bis zu 6 Kohlenstoffatomen, Alkoxycarbonylalkoxygruppen mit bis zu 8 Kohlenstoffatomen, Carbamoyl- und Alkylamidogruppen mit bis zu 6 Kohlenstoffatomen, Imidazolylgruppen, und Phenyl- und Phenoxygruppen, die gegebenenfalls durch ein oder mehrere Halogenatome oder Halogenalkylgruppen mit bis zu 6 Kohlenstoffatomen substituiert sind, oder durch einen Thiadiazolring disubstituiert sind, bedeutet; und

$R^1$ und $R^2$ jeweils ein Wasserstoffatom darstellen oder gemeinsam eine einfache Kohlenstoff-Kohlenstoff-Binding darstellen;

mit der Maßgabe, daß dann, wenn X für NH steht und $R^1$ und $R^2$ beide ein Wasserstoffatom bedeuten, R nicht eine Phenylgruppe ist.

**2.** Zusammensetzung nach Anspruch 1, worin X ein Sauerstoff- oder Schwefelatom darstellt.

**3.** Zusammensetzung nach Anspruch 1 oder 2, worin R eine Alkylgruppe mit 1 bis 4 Kohlenstoffatomen; eine Allylgruppe; eine Pyridyl-, Methylpyridyl-, Nitropyridyl-, Methylpyrimidinyl-, Pyrazinyl-, Chinolyl-, Indolyl-, Benzimidazolyl- oder Benzoxazolylgruppe; eine Naphthylgruppe, die gegebenenfalls durch ein Bromatom substituiert ist; oder eine Phenylgruppe darstellt, die gegebenenfalls durch 1 bis 3 Substituenten, ausgewählt unter Fluor-, Chlor- und Bromatomen, Carboxyl-, Hydroxyl-, Amino-, Nitro- und Cyanogruppen, Methyl-, Propyl-, Trifluormethyl-, Cyclohexyl-, Formyl-, Acetyl-, Propionyl-, Methoxy-, Methylthio-, Methylsulfinyl-, Methylsulfonyl-, Methoxycarbonylethoxy-, Acetamido-, Imidazolyl- oder Phenylgruppen und durch ein Chloratom und eine Trifluormethylgruppe substituierte Phenoxygruppen substituiert ist oder durch einen Thiadiazolring vicinal disubstituiert ist; und $R^1$ und $R^2$ zusammen eine einfache Kohlenstoff-Kohlenstoffbindung darstellen.

**4.** Zusammensetzung nach Anspruch 1 oder 2, worin R eine Phenylgruppe darstellt, die durch 1 oder 2 Substituenten,ausgewählt unter Chloratomen, Methylgruppen und Trifluormethylgruppen, substituiert ist und $R^1$ und $R^2$ jeweils ein Wasserstoffatom bedeuten.

**5.** Zusammensetzung nach einem der Ansprüche 1 bis 4, die wenigstens zwei Träger umfaßt, von denen wenigstens einer ein oberflächenaktives Mittel ist.

**6.** Verfahren zur Herstellung einer fungiziden Zusammensetzung, wie in einem der Ansprüche 1 bis 4 definiert, welches ein Zusammenbringen einer Thiazinonverbindung, wie in Anspruch 1 definiert, mit wenigstens einem Träger umfaßt.

**7.** Thiazinonverbindung der allgemeinen Formel I, wie in Anspruch 1 dargestellt, worin X, R, $R^1$ und $R^2$ wie in Anspruch 1 definiert sind, mit der Maßgabe, daß dann, wenn X für NH steht, R nicht eine tert.Butylgruppe oder 2-Methoxyphenylgruppe bedeutet; daß dann, wenn X für NH steht und $R^1$ und $R^2$ beide ein Wasserstoffatom darstellen, R nicht eine Hexyl-, 2-Methylphenyl-, 4-Methylphenyl-, 4-Chlorphenyl-, 4-Nitrophenyl- oder Naphthylgruppe bedeutet; daß dann, wenn X für NH steht und $R^1$ und $R^2$ zusammen eine einfache Kohlenstoff-Kohlenstoffbindung darstellen, R nicht eine Phenylgruppe ist; und daß dann, wenn X für 0, S oder NH steht und $R^1$ und $R^2$ zusammen eine einfache Kohlenstoff-Kohlenstoffbindung darstellen, R nicht eine Methylgruppe ist.

**8.** Verfahren zur Herstellung einer Thiazinonverbindung der allgemeinen Formel I, wie in Anspruch 7 definiert, welches Verfahren ein Umsetzen einer Verbindung der allgemeinen Formel II

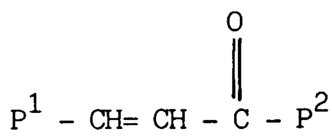

II

mit einer Verbindung der allgemeinen Formel III

RX - Q III,

worin X und R wie oben definiert sind, umfaßt, worin, wenn $R^1$ und $R^2$ im resultierenden Produkt der Formel I jeweils ein Wasserstoffatom darstellen, $P^1$ ein Wasserstoffatom bedeutet, $P^2$ ein Halogenatom bedeutet und Q eine Gruppe

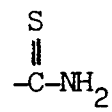

darstellt; und, wenn $R^1$ und $R^2$ im resultierenden Produkt der Formel I zusammen eine einfache Kohlenstoff-Kohlenstoffbindung darstellen, $P^1$ und $P^2$ zusammen eine Gruppe

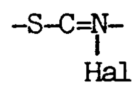

bedeuten, worin Hal ein Halogentaom darstellt und Q ein Wasserstoffatom bedeutet.

**9.** Verfahren nach Anspruch 8, worin die Umsetzung in einem inerten organischen Lösungsmittel in Anwesenheit einer Base ausgeführt wird.

**10.** Thiazinonverbindung nach Anspruch 7, wann immer sie nach einem Verfahren nach Anspruch 8 oder Anspruch 9 hergestellt ist.

**11.** Verfahren zur Bekämpfung von Pilzen an einem Ort, worin der Ort Pflanzen, die einem Pilzangriff unterliegen oder unterlagen, Samen solcher Pflanzen oder das Medium umfaßt, worin die Pflanzen wachsen oder gezogen werden sollen, gekennzeichnet durch Behandeln des Ortes mit einer fungizid wirksamen Menge einer Zusammensetzung, wie in einem der Ansprüche 1 bis 5 beansprucht, oder mit einer Verbindung, wie in Anspruch 7 oder Anspruch 10 beansprucht.

**12.** Verwendung einer Verbindung der Formel I, wie in einem der Ansprüche 1 bis 4, 7 und 10 definiert, als Fungizid.

## Revendications

**1.** Une composition fongicide qui renferme un support conjointement avec, comme ingrédient actif, un composé de thiazinone présentant la formule générale I :

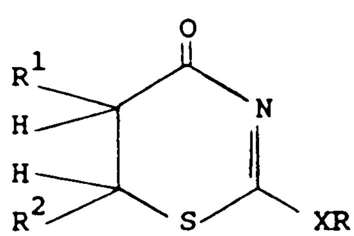

I

dans laquelle X représente un atome d'oxygène ou de soufre ou bien le groupe NH ; R représente un groupe alkyle ou alcényle possédant jusqu'à 6 atomes de carbone ; un groupe pyridiyle, pyrimidinyle, pyrazinyle, quinolyle, indolyle, benzimidazolyle, benzoxazolyle ou coumarinyle substitué, le cas échéant, par un atome d'halogène, un groupe nitro ou un groupe alkyle présentant jusqu'à 6 atomes de carbone ; un groupe naphtyle ou phényle substitué, le cas échéant, par un ou plus d'un substituant choisi parmi les atomes d'halogène, les groupes carboxyle, cyano, hydroxyle, amino et nitro, les groupes alkyle et haloalkyle présentant jusqu'à 6 atomes de carbone, les groupes cycloalkyle de 3 à 8 atomes de carbone, les groupes alkanoyle présentant jusqu'à 6 atomes de carbone, les groupes alkoxy et alkylthio présentant jusqu'à 6 atomes de carbone, les groupes alkylsulfinyle et alkylsulfonyle présentant jusqu'à 6 atomes de carbone, les groupes alkoxycarbonylalkoxy présentant jusqu'à 8 atomes de carbone, les groupes carbamoyle et alkylamido présentant jusqu'à 6 atomes de carbone, les groupes imidazolyle, les groupes phényle et phénoxy substitués, le cas échéant, par un ou plus d'un atome d'halogène ou les groupes haloalkyle présentant jusqu'à 6 atomes de carbone, ou bien disubstitué, de façon adjacente, par un cycle thiadiazole ; et $R^1$ et $R^2$ représentent chacun un atome d'hydrogène ou représentent conjointement une simple liaison carbone-carbone, sous la condition que, lorsque X est NH et $R^1$ et $R^2$ représentent conjointement un atome d'hydrogène, R alors n'est pas un groupe phényle.

**2.** Une composition telle que revendiquée dans la revendication 1, par laquelle X est un atome d'oxygène ou de soufre.

**3.** Une composition telle que revendiquée dans la revendication 1 ou la revendication 2, R représente un groupe alkyle de 1 à 4 atomes de carbone; un groupe allyle ; un groupe pyridyle, méthylpyridyle, nitropyridyle, méthylpyrimidinyle, pyrazinyle, quinolyle, indolyle, benzimidazolyle ou benzoxazolyle ; un groupe naphtyle, le cas échéant, substitué par un atome de brome ; un groupe phényle substitué, le cas échéant, par 1 à 3 substituants choisis parmi les atomes de fluor, de chlore et de brome, des groupes carboxyle, hydroxyle, amino, nitro et cyano, des groupes méthyle, propyle, trifluorométhyle, cyclohexyle, formyle, acétyle, propionyle, méthoxy, méthylthio, méthylsulfinyle, méthylsulfonyle, méthoxycarbonyléthoxy, acétamido, imidazolyle ou phényle, des groupes phénoxy substitués par un atome de chlore et un groupe trifluorométhyle, disubstitué, de façon adjacente, par un cycle thiadiazole; et $R^1$ et $R^2$ représentent ensemble une simple liaison carbone-carbone.

**4.** Une composition telle que revendiquée dans la revendication 1 ou 2, dans laquelle R représente un groupe phényle substitué par 1 ou 2 substituant choisi parmi les atomes de chlore, les groupes méthyle et les groupes trifluorométhyle et $R^1$ et $R^2$ représentent chacun un atome d'hydrogène.

**5.** Une composition telle que revendiquée dans l'une quelconque des revendications de 1 à 4, qui comporte au moins deux supports, dont au moins l'un d'entre eux est un agent tensioactif.

**6.** Une méthode pour préparer une composition fongicide telle que définie dans l'une quelconque des revendications de 1 à 4, méthode selon laquelle on amène un composé de thiazinone tel que défini dans la revendication 1 en association avec au moins un support.

**7.** Un composé de thiazinone de la formule générale I tel que revendiqué dans la revendication 1, dans lequel dans laquelle X, R, $R^1$ et $R^2$ sont tels que définis dans la revendication 1, sous la condition que, lorsque X est NH, alors R n'est pas un groupe tert-butyle ni 2-méthoxyphényle ; lorsque X est NH et $R^1$ et $R^2$ représentent tous les deux un atome d'hydrogène, alors R n'est pas un groupe hexyle, 2-méthylphényle, 4-méthylphényle, 4-chlorophényle, 4-nitrophényle ou naphtyle ; lorsque X est NH et $R^1$ et $R^2$ représentent ensemble une simple liaison carbone-carbone, alors R n'est pas un groupe phényle; et lorsque X est 0, S ou NH et $R^1$ et $R^2$ représentent conjointement une simple liaison carbone-carbone, alors R n'est pas un groupe méthyle.

**8.** Un procédé pour la préparation d'un composé thiazinone de formule I tel que défini dans le paragraphe précédent, procédé dans lequel on fait réagir un composé de formule générale II

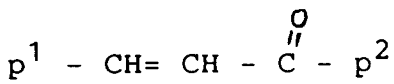

II

avec un composé de formule générale III

RX - Q III

dans laquelle X et R sont tels que définis dans la revendication 7, lorsque $R^1$ et $R^2$ dans le produit résultant de formule I représentent chacun un atome d'hydrogène, $p^1$ représente un atome d'hydrogène, $p^2$ représente un atome d'halogène et Q représente un groupe

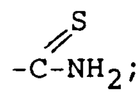

et lorsque $R^1$ et $R^2$ dans le produit résultant de formule I représentent ensemble une simple liaison carbone-carbone, $p^1$ et $p^2$ représentent conjointement un groupe

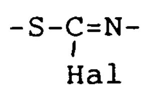

dans lequel Hal représente un atome d'halogène et Q représente un atome d'hydrogène.

**9.** Un procédé tel que revendiqué dans la revendication 8, dans lequel la réaction est mise en oeuvre dans un solvant organique inerte en présence d'une base.

**10.** Un composé de thiazinone tel que revendiqué dans la revendication 7, chaque fois qu'il est préparé par un procédé tel que revendiqué dans la revendication 8 ou la revendication 9.

**11.** Une méthode pour combattre les mycètes en un endroit, dans laquelle l'endroit comporte des plants soumis à ou susceptibles d'être soumis à une attaque fongique, des graines de ces plantes ou le milieu dans lequel ces plantes croissent ou doivent se développer, caractérisée en ce que l'on traite l'endroit avec une quantité efficace du point de vue fongicide d'une composition telle que revendiquée dans l'une quelconque des revendications 1 à 5 ou bien un composé tel que revendiqué dans la revendication 7 ou la revendication 10.

**12.** L'utilisation comme fongicide d'un composé de formule I tel que défini dans l'une quelconque des revendications 1 à 4, 7 et 10.